# EUROPÄISCHE PATENTSCHRIFT

(11) **EP 1 216 277 B1**
(45) Veröffentlichungstag und Bekanntmachung des Hinweises auf die Patenterteilung: **16.11.2005**
(21) Anmeldenummer: 00953195.5
(22) Anmeldetag: 23.08.2000
(51) Int. Cl.: C09C 1/00, C09D 7/12, C09D 11/00, C04B 33/14, A61K 7/00, C08K 3/00, C03C 1/04

(54) **GLANZPIGMENTE MIT ABSORBIERENDER, NIEDRIGBRECHENDER BESCHICHTUNG**
GLOSS PIGMENTS COMPRISING AN ABSORBENT LOW-REFRACTIVE COATING
PIGMENTS BRILLANTS A REVETEMENT ABSORBANT A FAIBLE INDICE DE REFRACTION

(30) Priorität: 31.08.1999 DE 19941253
(43) Veröffentlichungstag der Anmeldung: 26.06.2002
(73) Patentinhaber: BASF Aktiengesellschaft, 67056 Ludwigshafen (DE)
(72) Erfinder: SCHMID, Raimund, D-67435 Neustadt (DE); BÖHM, Arno, D-68305 Mannheim (DE); SEEGER, Oliver, D-68163 Mannheim (DE); MRONGA, Norbert, D-69221 Dossenheim (DE); CLEMENS, Thorsten, D-67126 Hochdorf-Assenheim (DE)
(86) Internationale Anmeldenummer: PCT/EP2000/008217
(87) Internationale Veröffentlichungsnummer: WO 2001/016236

(56) Entgegenhaltungen:
- EP-A- 0 753 545
- EP-A- 0 940 451
- EP-A- 0 959 109
- EP-A- 1 028 146
- WO-A-93/08237
- DE-A- 4 140 295
- DE-A- 4 437 753
- "Perlglanzpigmente", Verlag Moderne Industrie, Maisch und Weigand, 1992, S. 40

## Beschreibung

Die vorliegende Erfindung betrifft neue Glanzpigmente auf der Basis von mehrfach beschichteten, reflektierenden, plättchenförmigen Substraten, die metallische oder hochbrechende nichtmetallische Plättchen mit einem Brechungsindex ≥ 2, die jeweils bereits mit einer hochbrechenden Schicht belegt sein können, oder niedrigbrechende nichtmetallische Plättchen, die bereits mit einer hochbrechenden Schicht belegt sind, umfassen und mindestens ein Schichtpaket aus
A) zunächst einer niedrigbrechenden, sichtbares Licht selektiv absorbierenden Beschichtung mit einem Brechungsindex ≤ 1,8 und
B) anschließend einer reflektierenden, für sichtbares Licht zumindest teilweise durchlässigen Beschichtung
   sowie gewünschtenfalls zusätzlich
C) eine äußere Schutzschicht
aufweisen.

Weiterhin betrifft die Erfindung die Verwendung dieser Glanzpigmente zum Einfärben von Lacken, Druckfarben, Tinten, Kunststoffen, Gläsern, keramischen Produkten und Zubereitungen der dekorativen Kosmetik.

Glanz- oder Effektpigmente werden in vielen Bereichen der Technik eingesetzt, beispielsweise in Automobillacken, in der dekorativen Beschichtung, der Kunststoffeinfärbung, in Anstrichfarben, Druckfarben, insbesondere Sicherheitsdruckfarben, sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Von besonderem Interesse sind goniochromatische Glanzpigmente, die einen winkelabhängigen Farbwechsel zwischen mehreren intensiven Interferenzfarben und damit ein attraktives Farbenspiel zeigen. Diese Pigmente weisen typischerweise einen lichtreflektierenden plättchenförmigen Kern auf, der im Wechsel mit niedrigbrechenden und hochbrechenden Schichten belegt ist.

Eine Übersicht über goniochromatische Glanzpigmente gibt European Coatings Journal 7-8, Seite 702-705 (1997). Einzelheiten zu den dort beschriebenen sowie weiteren goniochromatischen Glanzpigmenten sind in den US-A-3 438 796, US-A-4 434 010, EP-A-668 329, WO-A-96/34917, EP-A-708 154, EP-A-753 545 und DE-A-197 46 067 sowie den älteren deutschen Patentanmeldungen 198 08 657.1 und 198 22 046.4 beschrieben.

Eine Übersicht über goniochromatische Glanzpigmente gibt European Coatings Journal 7-8, Seite 702-705 (1997). Einzelheiten zu den dort beschriebenen sowie weiteren goniochromatischen Glanzpigmenten sind in den US-A-3 438 796, US-A-4 434 010, EP-A-668 329, WO-A-96/34917, EP-A-708 154, EP-A-753 545 und DE-A-197 46 067 sowie den älteren deutschen Patentanmeldungen 198 08 657.1 und 198 22 046.4 beschrieben.

Allen bekannten goniochromatischen Glanzpigmenten ist eine farblose, niedrigbrechende, oft als dielektrisch bezeichnete Schicht gemeinsam, die für den winkelabhängigen Farbeindruck der Pigmente verantwortlich ist und deren zarte Interferenzfarben durch die Kombination mit hochbrechenden und gegebenenfalls absorbierenden Schichten verstärkt werden. Bei der Applikation wird das Farbenspiel dieser Pigmente üblicherweise durch Zumischen anderer Farbmittel zur Anpassung an die jeweiligen koloristischen Anforderungen und den persönlichen Geschmack des Anwenders modifiziert. Da Farbempfinden und persönlicher Geschmack sehr individuell sind, besteht jedoch ständig ein Bedarf nach neuen Effekten, der nicht immer durch Kombination bekannter Farbmittel befriedigt werden kann.

In der nicht vorveröffentlichten EP-A-1 028 146 sind goniochromatische Glanzpigmente beschrieben, die eine farbige niedrigbrechende Beschichtung aufweisen, jedoch nicht mit einer auf diese Beschichtung folgenden reflektierenden Schicht belegt sind.

Der Erfindung lag die Aufgabe zugrunde, weitere goniochromatische Glanzpigmente bereitzustellen, die neue koloristische Effekte zeigen und sich durch vorteilhafte Anwendungseigenschaften auszeichnen.

Demgemäß wurden die eingangs definierten goniochromatischen Glanzpigmente gefunden.

Weiterhin wurde die Verwendung der erfindungsgemäßen Glanzpigmente zur Einfärbung von Lacken, Druckfarben, Tinten, Kunststoffen, Gläsern, keramischen Produkten und Zubereitungen der dekorativen Kosmetik gefunden.

Die erfindungsgemäßen Glanzpigmente basieren auf mehrfach beschichteten, reflektierenden, plättchenförmigen Substraten, die eine niedrigbrechende Beschichtung (A), die sichtbares Licht selektiv absorbiert, in Kombination mit einer reflektierenden, für sichtbares Licht zumindest teilweise durchlässigen Beschichtung (B) aufweisen.

Als Substratmaterial eignen sich für die erfindungsgemäßen Glanzpigmente alle plättchenförmigen Materialien, die senkrecht auftreffendes Licht ganz oder teilweise (üblicherweise zu mindestens 10%) reflektieren. In der Regel sind diese Materialien hochbrechend und haben üblicherweise einen Brechungsindex von in der Regel ≥ 2, vorzugsweise ≥ 2 , 4 , sie können opak, semiopak oder Transparenz und in Reflexion bzw. Transmission auch farbig sein.

Eine Gruppe geeigneter Substratmaterialien sind Mecallplättchen. Es kommen alle für Metallefrektpigmente bekannten Metalle und Legierungen, z.B. Stahl, Kupfer und seine Legierungen wie Messing und Bronzen und insbesondere Aluminium und seine Legierungen wie Aluminiumbronze, in Betracht. Bevorzugt sind Aluminiumflakes, die in einfacher Weise durch Herausstanzen aus Aluminiumfolie oder nach gängigen Verdüsungs- und Mahltechniken herzustellen sind, wobei handelsübliche Produkte eingesetzt werden können, deren Oberfläche jedoch weitgehend frei von Festen oder ähnlichen Belegmitteln sein sollte und passiviert, d.h. insbesondere gegenüber Wasser stabilisiert, sein kann.

Die metallischen Substratteilchen können gewünschtenfalls bereits mit hochbrechenden Metallverbindungen wie hochbrechendem Metalloxid, Mecallnitrid oder Metallsulfid, insbesondere z.B. Eisen- oder Titanoxid, belegt sein und daher durch Interferenzeffekte und gegebenenfalls Absorption bereits eine (schwache) Eigenfarbe besitzen (Paliocrom®, BASF). Diese Beschichtung sollte jedoch nicht zu dick sein (etwa 5 bis 150 nm), damit die Substratteilchen ihre metallische Koloristik behalten. Weiterhin können die metallischen Substratteilchen auch mit magnetischen Materialien wie Eisen, Kobalt, Nickel oder γ-Eisez(III)oxid belegt und damit magnetisierbar sein.

Eine weitere Gruppe geeigneter Substratmaterialien sind nichtmetallische Plättchen, die "von sich aus" hochbrechend sind oder die "von sich aus" nur niedrigbrechend und deshalb mit einer hochbrechenden Beschichtung versehen sind.

Beispiele für besonders geeignete von sich aus hochbrechende Materialien sind selektiv oder nichtselektiv absorbierende Materialien, z.B. plättchenförmige Metalloxide, -sulfide und -nitride wie vor allem plättchenförmiges (semiopakes) α-Eisen(III)oxid (α-Fe₂O₃, Hämatit), das mit Silicium (EP-A-14 382), Aluminium (EP-A-68 311) oder Aluminium und Mangan (EP-A-265 820) dotiert sein kann (z.B. Paliocrom® Kupfer L3000, BASF; AM200, Titan Kogyo), plättchenförmiges (opakes) Eisen(II/III)oxid (Fe₃O₄, Magnetit), Molybdänsulfid-, Bornitrid- und Graphitplättchen. Ebenfalls geeignet sind nichtabsorbierende (farblose), transparente Materialien wie plättchenförmiges Bismutoxychlorid, Titandioxid- und Zirkondioxidplättchen.

Beispiele für besonders geeignete von sich aus nur niedrigbrechende Materialien sind vor allem silikatische Plättchen wie insbesondere helle bzw. weiße Glimmer, vorzugsweise naß vermahlener Muskovit, aber auch andere natürliche Glimmer, z.B. Phlogopit und Biotit, künstliche Glimmer, Talk- und Glasschuppen und Siliciumdioxidplättchen.

Als hochbrechende Beschichtung für diese niedrigbrechenden Materialien eignen sich insbesondere hochbrechende Metalloxide, Metallnitride und Metallsulfide wie Titan-, Zirkon-, Zink- und Zinnoxid, Bismutoxychlorid, Eisenoxide, Chromoxid und Ilmenit sowie reduzierte, Titan mit Oxidationszahlen von < 4 bis 2 enthaltende Titanverbindungen wie Ti₃O₅, Ti₂O₃, TiO, Titanoxynitride und TiN, die bei Reduktion titandioxidbeschichteter Substrate mit Ammoniak, Wasserstoff und/oder Kohlenwasserstoffen entstehen. Bevorzugt sind hierbei neben Ilmenit insbesondere Titandioxid und seine Reduktionsprodukte sowie Eisen(III)oxid.

Derartig beschichtete Glimmerpigmente sind ebenfalls im Handel erhältlich (Paliocrom, BASF; Iriodin®, Merck; Mearlin®, Mearl).

Übliche geometrische Schichtdicken für diese hochbrechenden Beschichtungen liegen im Bereich von etwa 10 bis 300 nm, insbesondere von 20 bis 200 nm.

Die Größe der Substratteilchen kann auf den jeweiligen Anwendungszweck abgestimmt werden. In der Regel haben die Plättchen mittlere größte Durchmesser von etwa 1 bis 200 µm, insbesondere 5 bis 100 µm, und Dicken von etwa 0,1 bis 1 µm, insbesondere um etwa 0,5 µm bei metallischen und um etwa 0,3 µm bei nichtmetallischen Substraten. Ihre spezifische freie Oberfläche (BET) beträgt üblicherweise 1 bis 15 m²/g, insbesondere 0,1 bis 5 m²/g bei metallischen und 1 bis 12 m²/g bei nichtmetallischen Substraten.

Die erfindungsgemäßen Glanzpigmente weisen eine niedrigbrechende, sichtbares Licht selektiv absorbierende Beschichtung (A) in Kombination mit einer reflektierenden, für sichtbares Licht zumindest teilweise durchlässigen Beschichtung (B) auf. Sie können mehrere gleiche oder verschiedene Kombinationen (Schichtpakete) (A) + (B) enthalten, bevorzugt ist aber die Belegung mit nur einem Schichtpaket (A) + (B).

Die erfindungsgemäße Beschichtung (A) ist vorzugsweise aus einem farblosen niedrigbrechenden Material aufgebaut, in das ein oder mehrere selektiv absorbierende Farbmittel eingelagert sind.

Das Schichtmaterial (A) und dementsprechend auch die Beschichtung (A) hat einen Brechungsindex n ≤ 1,8, insbesondere ≤ 1,6.

Als Schichtmaterial (A) sind dabei prinzipiell alle niedrigbrechenden farblosen Substanzen, die filmartig und dauerhaft auf die Substratteilchen aufgebracht werden können, geeignet.

Besonders geeignet sind z.B. Metalloxide und Metalloxidhydrate wie Siliciumoxid, Siliciumoxidhydrat, Aluminiumoxid, Aluminiumoxidhydrat, Aluminiumhydroxid und deren Mischungen, wobei Siliciumoxid(hydrat) bevorzugt ist.

Die bevorzugte erfindungsgemäße Beschichtung (A) enthält eingelagertes selektiv absorbierendes Farbmittel. Dieses Farbmittel kann im wesentlichen homogen in der Beschichtung (A) verteilt oder in einem Teilbereich der Beschichtung (A), z.B. im unteren oder oberen Teil oder in der Mitte der Schicht (A), angereichert sein bzw. kann die Beschichtung (A) einen Konzentrationsgradienten bezüglich des Farbmittels aufweisen. Bevorzugt ist das Farbmittel im unteren, substratnahen Bereich der Beschichtung (A) angereichert.

Als Farbmittel sind dabei alle Farbstoffe und Pigmente geeignet, die dauerhaft in die Schicht (A) eingelagert werden können und deren Brechungsindex vorzugsweise nicht wesentlich über dem Brechungsindex des Schichtmaterials (A) liegt. Prinzipiell können auch höherbrechende Farbmittel verwendet werden, jedoch sollte ihr Anteil an der Beschichtung (A) dann nicht so groß sein, daß sich der Brechungsindex der Beschichtung (A) merklich erhöht und die Goniochromatizität der erfindungsgemäßen Glanzpigmente verringert.

Neben niedrigbrechenden anorganischen Pigmenten wie Cyanoferraten (z.B. K₃[Fe(cN)₆], K[Fe(II)Fe(III) (CN)₆],
Fe(III)[Fe(II)Fe(III) (CN)₆]), Granaten (z.B. Ca₃Cr₂Si₃O₁₂), Phosphaten (insbesondere Cobaltphosphaten, z.B. Co₃(PO₄)₂, CoLiPO₄), Boraten (insbesondere Cobaltboraten, z.B. (Co,Mg)₂B₂O₅) und Ultramarin (z.B. Na₄[Al₃Si₃O₁₂]S₃) eignen sich vor allem organische Pigmente, da ihre Wechselwirkung mit Licht insbesondere auf Absorption beruht. Beispielhaft seien hier folgende Pigmentklassen aufgeführt: Azo-, Disazo-, Anthanthron-, Anthrachinon-, Anthrapyrimidin-, Chinacridon-, Chinophthalon-, Diketopyrrolopyrrol-, Dioxazin-, Flavanthron-, Indanthron-, Isoindolin-, Isoindolinon-, Isoviolanthron-, Metallkomplex-, Perinon-, Perylen-, Phthalocyanin-, Pyranthron-, Thioindigo- und Triarylcarboniumpigmente.

Besonders geeignet sind Farbstoffe, da sie im Gegensatz zu Pigmenten nicht als Streuzentrum wirken. Im Prinzip können alle Farbstoffe eingesetzt werden, bevorzugt sind solche Farbstoffe, die in Wasser und/oder in mit Wasser mischbaren organischen Lösungsmitteln, z.B. Alkoholen, löslich sind und daher unter den üblicherweise verwendeten Reaktionsbedingungen zur Herstellung der Beschichtung (A) vorteilhaft in diese Schicht eingebaut werden können.

Ganz besonders geeignet sind Farbstoffe, die eine große Affinität zum Schichtmaterial (A) aufweisen. Bei saurem Schichtmaterial (A), z.B. Siliciumdioxid, sind daher kationische (basische) Farbstoffe bevorzugt, bei basischem Schichtmaterial (A), z.B. Aluminiumoxid, sind anionische Farbstoffe (Säurefarbstoffe) bevorzugt. In amphoteren Schichtmaterialien sind beide Farbstoffklassen einsetzbar.

Kationische Farbstoffe tragen eine positive Ladung, die über das Farbstoffmolekül delokalisiert oder an einem Heteroatom, wie vor allem einem Stickstoffatom (quaternäres Ammoniumion), aber auch einem Sauerstoff-, Schwefel- oder Phosphoratom, lokalisiert sein kann. Das salzbildende Gegenion ist meistens das farblose Anion einer anorganischen oder organischen Säure geringen Molekulargewichts.

Als geeignete kationische Farbstoffe mit delokalisierter positiver Ladung seien z.B. genannt:
- Methinfarbstoffe (vinyloge Amidiniumsalze):
   Enaminfarbstoffe (ein terminales Stickstoffatom der Methinkette ist Bestandteil eines heterocyclischen Rings, das andere Stickstoffatom ist direkt an die Mechinkette gebunden);
   Cyaninfarbstoffe (beide terminalen Stickstoffatome sind jeweils Bestandteil eines heterocyclischen Rings);
   Mono- oder Diazamethinfarbstoffe (ein oder zwei Stickstoffatome ersetzen zusätzlich Kohlenstoffatome in der Methinkette);
   Diazacyaninfarbstoffe (kationische Azofarbstoffe)
- Di- und Triarylmethanfarbstoffe:
   Monomethinfarbstoffe mit zwei oder drei terminalen Aryl-(insbesondere Phenyl-)Resten, bei denen mindestens einer, bevorzugt aber zwei bzw. drei Arylreste in para-Stellung zum Methinkohlenstoffatom durch eine elektronendonierende Gruppe (meistens eine Dialkylaminogrupoe) substituiert sind; z.B.
   Malachitgrün und Kristallviolett
- Xanthenfarbstoffe:
   Triphenylmethanfarbstoffe mit einem zwei Phenylringe verbrückenden Sauerstoffatom; z.B. Rhodamin B
- Azinfarbstoffe:
   Phenazinfarbstoffe (dibenzokondensiertes Pyrazin);
   Phenoxazinfarbstoffe (dibenzokondensiertes 1,4-Oxazin);
   Phenothiazinfarbstoffe (dibenzokondensiertes 1,4-Thiazin), z.3. Methylenblau.

Als kationische Farbstoffe mit lokalisierter positiver Ladung eignen sich insbesondere solche Farbstoffe, die durch Formel I beschrieben werden können:

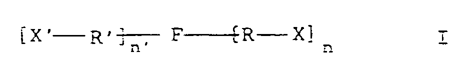

Dabei haben die Variablen folgende Bedeutung:
- F: den (n+n')-wertigen Rest eines Chromophors aus der Gruppe der Anthanthrone, Anthrachinone, Anthrapyrimidine, Mono-, Dis- und Polyazofarbstoffe, Chinacridone, Chinophthalone, Diketopyrrolopyrrole, Dioxazine und Bisdioxazine, Flavanthrone, Indanthrone, Isoindoline, Isoindolinone, Naphthalimide, Perinone, Perylene, Terrylene und Quaterrylene, Phthalocyanine, Porphyrine, Pyranthrone, Rhodamine, Violanthrone und Isoviolantnrone, Thioindigofarbstoffe und Xanthene;
- R, R': unabhängig voneinander einen C₁-C₁₀-Alkylenrest, dessen Kohlenstoffkette durch Sauerstoffatome in Etherfunktion unterbrochen sein kann, wobei Ethylen und insbesondere Methylen bevorzugt sind, oder einen Arylen-, Hetarylen-, Aralkylen- oder Hetaralkylenrest, wobei. Phenylen, Naphthylen, Pyridylen, Methylenphenylen und Ethylenphenylen bevorzugt sind;
- X, X': unabhängig voneinander einen einwertigen positiv geladenen Rest, bevorzugt
einen Rest der Formel Ia

― N^{⊕} R¹R²R³ Ia

wobei R¹, R² und R³ unabhängig voneinander C₁-C₁₀-Alkyl, C₁-C₁₀-Alkoxy, Aryl oder Hetaryl bedeuten, das jeweils ein- oder mehrfach durch Hydroxy oder Cyano substituiert sein kann, wobei zwei der Reste R¹, R² und R³ auch mit dem Stickstoffatom unter Ausbildung eines 5- bis 7-gliedrigen, über das stickstoffatom gebundenen, gesättigten Rings verbunden sein können,
einen Rest der Formel Ib 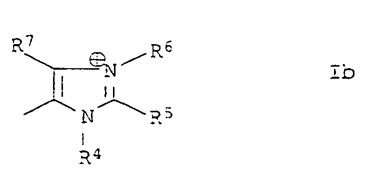 wobei R⁴, R⁵, R⁶ und R⁷ unabhängig voneinander Wasserstoff, C₁-C₄-Alkyl, insbesondere Methyl oder Ethyl, C₁-C₄-Hydroxyalkyl, insbesondere Hydroxymethyl oder Hydroxyethyl, bedeuten, wobei solche Reste Ib bevorzugt sind, in der R⁴ und R⁶ unabhängig voneinander Methyl, Hydroxyethyl oder Wasserstoff, R⁵ Wasserstoff und R⁷ Methyl oder Wasserstoff bedeuten, oder
einen Rest der Formeln Ic 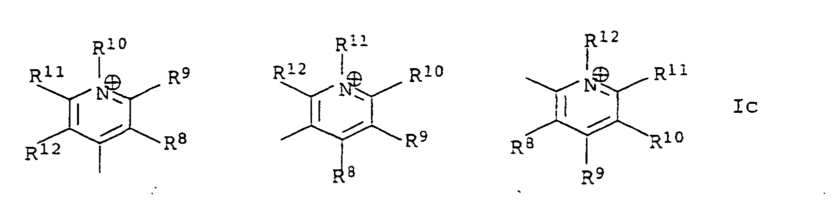 wobei R⁸, R⁹, R¹⁰, R¹¹ und R¹² unabhängig voneinander Wasserstoff, C₁-C₄-Alkyl und C₁-C₄-Alkoxy bedeuten;
- n, n': jeweils 0 bis 4, wobei (n+n') mindestens 1 ist.

Als farbloses Anion eignen sich dabei z.B. Halogenide, insbesondere Chlorid, Sulfat, Hydrogensulfat, Phosphat, Hydrogen- und Dihydrogenphosphat, Nitrat, Hydroxid, Formiat, Acetat, Pronionat und Benzoat.

Die kationischen Farbstoffe der Formel I sind hinlänglich bekannt. Imidazolylmethylierte Farbstoffe sind beispielsweise in den EP-A-335 237 und 643 108 beschrieben.

Als anionische Farbstoffe sind insbesondere solche Farbstoffe geeignet, die durch Formel II beschrieben werden können:

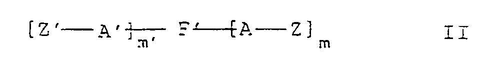

Dabei haben die Variablen folgende Bedeutung:
- F': den (m=m')-wertigen Rest eines Chromoohors aus der Gruppe der Anihanthrone, Anthrachinone, Anthrapyrimidine, Mono-, Dis- und Polyazofarbstoffe, Chinacridone, Chinophthalone, Diketopyrrolopyrrole, Dioxazine und Bisdioxazine, Flavanthrone, Indanthrone, Isoindoline, Isoindolinone, Naphthalimide, Perinone, Perylene, Terrylene und Quaterrylene, Phthalocyanine, Porphyrine, Pyranthrone, Rhodamine, Violanthrone und Isoviolanthrone, Thioindigofarbstoffe und Xanthene;
- A, A': unabhängig voneinander eine chenische Bindung, einen C₁-C₁₀-Alkylenrest, dessen Kohlenstoffkette durch Sauerstoffatome in Etherfunktion unterbrochen sein kann, wobei Ethylen und insbesondere Methylen bevorzugt sind, oder einen Arylen-, Hetarylen-, Aralkylen- oder Hetaralkylenrest, wobei Phenylen, Naphthylen, Pyridylen, Methylenphenylen und Ethylenphenylen bevorzugt sind;
- Z, Z': unabhängig voneinander einen einwertigen negativ geladenen Rest, bevorzugt einen Rest der Formeln IIa bis IIc

― SO₃ ^{⊖} IIa

―COO^{⊖} IIb

―O^{⊖} IIc
- m, m': jeweils 0 bis 6, wobei (m+m') mindestens 1 ist.

Das salzbildende Gegenion der anionischen Farbstoffe ist in der Regel ein farbloses Kation, z.B. ein Wasserstoffkation, Alkalimetallkation, insbesondere Lithium-, Natrium-, Kaliumkation, Erdalkalimetallkation, insbesondere Magnesium-, Calciumkation, oder Ammoniumion.

Die anionischen Farbstoffe der Formel II sind ebenfalls hinlänglich bekannt.

Die erfindungsgemäß bevorzugte Beschichtung (A) enthält in der Regel 0,1 bis 50 Gew.-%, bevorzugt 1 bis 40 Gew.-% und besonders bevorzugt 5 bis 25 Gew.-%, Farbmittel.

Die geometrische Schichtdicke der 3eschichtung (A) beträgt im allgemeinen 10 bis 800 nm, vorzugsweise 100 bis 600 nm. Da die Schicht (A) im wesentlichen die Interferenzfarben der erfindungsgemäßen Pigmente bestimmt, hat sie für ein ausgeprägtes Farbenspiel zeigende und daher auch bevorzugte Glanzpigmente, die nur ein Schichtpaket (A) + (B) aufweisen, eine geometrische Mindestschichtdicke von 100 nm, vorzugsweise von 200 nm. Sind mehrere (z.B. 2, 3 oder 4) Schichtpakete (A) + (3) vorhanden, dann liegt die geometrische Schichtdicke von (A) bevorzugt bei 50 bis 200 nm.

Mit zunehmender Dicke der Beschichtung (A) nimmt die winkelabhängigkeit des Farbtons der erfindungsgemäßen Glanzpigmente zu. Im Gegensatz zu den bekannten Glanzpigmenten wird der Farbeindruck der erfindungsgemäßen Glanzpigmente nicht nur durch Interferenz sondern auch stark durch Absorption beeinflußt. Durch geeignete Wahl des Farbmittels kann das Farbenspiel der erfindungsgemäßen Glanzpigmente eindrucksvoll variiert werden. Um den Farbeindruck zu verstärken und auch in Anwendungsmeäien mit niedrigem Brechungsindex, z.B. Lacken, deutlich sichtbar zu machen, sind die erfindungsgemäßen Glanzpigmente zusätzlich mit der Beschichtung (B) versehen.

Die reflektierende Beschichtung (B) der erfindungsgemäßen Glanzpigmente muß für sichtbares Licht zumindest teilweise durchlässig sein, d.h. in der Regel mindestens 10%, bevorzugt mindestens 30%, des auftreffenden Lichts durchlassen.

Für die erfindungsgemäße Beschichtung (B) eignen sich sowohl hochbrechende Materialien, die sichtbares Licht nicht absorbieren, selektiv absorbieren oder nichtselektiv absorbieren, als auch niedrigbrechende, jedoch im sichtbarer- Wellenlängenbereich eine hohe Absorptionskonstante (in der Regel ≥ 4) aufweisende Substanzen, die selbstverständlich auch filmartig und dauerhaft abscheidbar sein müssen.

Als hochbrechende, für die Beschichtung (B) besonders geeignete Materialien seien beispielsweise Metalle und Metallverbindungen wie Metalloxide, Metallnitride und Metallsulfide und deren Mischungen genannt, die auch niedrigbrechende Substanzen in untergeordneter Menge enthalten können, solange der Brechungsindex n der Mischung sich nicht merklich erniedrigt, der in der Regel n ≥ 2, insbesondere ≥ 2,4, sein sollte.

Im einzelnen seien folgende Beispiele für besonders geeignete hochbrechenden Schichtmaterialien (B) genannt:
- nichtabsorbierende Materialien:
   Metalloxide wie Titandioxid, Titanoxidhydrat, Zirkoniumdioxid, Zirkoniumoxidhydrat, Zinndioxid, Zinnoxidhydrat, Zinkoxid, Zinkoxidhydrat und deren Mischungen, wobei Titandioxid und Titanoxidhydrat und deren Mischungen mit bis zu etwa 5 Gew.-% der anderen Metalloxide, insbesondere Zinndioxid, aber auch Siliciumdioxid, bevorzugt sind; Bismutoxychlorid; Metallsulfide wie Zinksulfid;
- selektiv absorbierende Materialien:
   Metalloxide und -nitride wie besonders bevorzugt Eisen(III)oxide (α- und γ-Fe₂O₃), Chrom(III)oxid, Titan(III)oxid und Titannitride (TiN und Titanoxynitride TiOₓN_{y}), wobei die niederen Titanoxide und -nitride in der Regel im Gemisch mit Titandioxid vorliegen, auch Bismutvanadat und Molydänsuboxide (Molybdänblau) sowie mit selektiv absorbierenden Farbmitteln "eingefärbte" farblose Metalloxidschichten, z.B. aus Titandioxid und Zirkondioxid, die mit selektiv absorbierenden Metallkationen dotiert oder mit einem Farbmittel enthaltenden Film überzogen sind;
- nichtselektiv absorbierende Materialien:
   Metalle, die durch Gasphasenzersetzung flüchtiger Metallverbindungen abgeschieden werden können, wie besonders bevorzugt Molybdän, bevorzugt Eisen, Wolfram und Chrom, auch Cobalt und Nickel sowie Gemische dieser Metalle, sowie Metalle, die naßchemisch durch Reduktion aus Metallsalzlösungen abgeschieden werden können, wie Silber, Kupfer, Gold, Palladium, Platin und Legierungen, z.B. NiP, NiB, NiCo, NiWP, CoP und AgAu; Metalloxide wie bevorzugt Magnetit (Fe₃O₄), auch Cobaltoxid (CoO, Co₃O₄) und Vanadiumoxid (VO₂, V₂O₃) sowie auch Mischungen dieser Oxide mit den Metallen, z.B. Magnetit/Eisen; Metallsulfide wie besonders bevorzugt Molybdänsulfid, bevorzuge Eisensulfid, Wolframsulfid und Chromsulfid, auch Cobaltsulfid und Nickelsulfid sowie Gemische dieser Sulfide wie MoS₂/WS₂ und vor allem auch Gemische dieser Sulfide mit dem jeweiligen Metall, z.B. MoS₂/Molybdän, und Gemische mit Oxiden des jeweiligen Metalls, z.B. MoS₂/Molybdänoxide; Kohlenstoff.

Als niedrigbrechende, jedoch stark absorbierende Schichtmaterialien (B) eignen sich vor allem nichtselektiv absorbierende Materialien wie Metalle, z.B. Aluminium.

Die geometrische Schichtdicke der Beschichtung (B) variiert in Abhängigkeit von den optischen Eigenschaften des ausgewählten Schichtmaterials und kann 1 bis etwa 500 nm betragen. Bevorzugte geometrischen Schichtdicken betragen bei hochbrechenden, nicht absorbierenden Materialien (B) 5 bis 50 nm, insbesondere 10 bis 40 nm, und bei hochbrechenden, selektiv absorbierenden Materialien (B) 1 bis 500 nm, insbesondere 10 bis 150 nm. Für hochbrechende, nichtselektiv absorbierende Materialien (B) liegt die geometrische Schichtdicke vorzugsweise im Bereich von 1 bis 100 nm, wobei für stark absorbierende Metall wie Molybdän und Chrom Schichtdicken von 1 bis 25 nm, für schwächer absorbierende Materialien wie Magnetit Schichtdicken von 10 bis 50 nm und für metallsulfidhaltige Materialien wie MoS₂-enthaltende Schichten Schichtdicken von 5 bis 20 nm besonders bevorzugt sind. Bei niedrigbrechenden, stark absorbierenden Materialien (B) beträgt die geometrische Schichtdicke schließlich bevorzugt 1 bis 25 nm, besonders bevorzugt 5 bis 20 nm.

Enthalten die erfindungsgemäßen Glanzpigmente mehrere Schichtpakete (A) + (B), so erniedrigt sich die Schichtdicke der Beschichtungen (B) üblicherweise um etwa 50 bis 75 %.

Durch die Beschichtung (B) wird das Farbenspiel der mit der Beschichtung (A) belegten Glanzpigmente verstärkt, gegebenenfalls (z.B. durch selektive Absorption) modifiziert und in allen Anwendungsmedien deutlich sichtbar gemacht. Wie bereits dargelegt, ist das Farbenspiel der mit (A) (und (B)) belegten Glanzpigmente gezielt durch die Auswahl des in die Beschichtung (A) eingebauten Farbmittels variierbar.

Wird beispielsweise ein plättchenförmiges Aluminiumpigment mit einer ca. 350 nm dicken Siliciumdioxidschicht und einer semitransparenten Molybdänschicht (B) belegt, so zeigt das applizierte Pigment in der Aufsicht ein kräftiges Grün, das bei steileren Betrachtungswinkeln nach Blau abkippt. Baut man in die Siliciumdioxidschicht ein blaues Farbmittel ein, so läßt sich der Farbtonbereich der blaustichigen Grüntöne im Farbenspiel des Pigments vorteilhaft ausweiten, das Pigment ist in Aufsicht türkis und bei steileren Betrachtungswinkeln blau.

Noch eindrucksvoller ist dieser Effekt z.B. bei einem ebenfalls mit einer ca. 350 nm dicken Siliciumdioxidschicht, jedoch anstatt mit der Molybdänschicht mit einer Eisen(III)oxidschicht (B) belegten Aluminiumpigment sichtbar. Vor dem Einbau des selektiv absorbierenden Farbmittels liegt ein grünlich goldenes Pigment vor, dessen Farbe bei steileren Betrachtungswinkeln nach unbunt abkippt. Der Einbau eines blauen Farbmittels in die Siliciumdioxidschicht bewirkt eine intensiv grüne Farbe in Aufsicht und bei steileren Betrachtungswinkeln ein Abkippen in blaustichiges Grün.

Schließlich können die erfindungsgemäßen Glanzpigmente noch eine äußere Schicht (C) aufweisen, die insbesondere zum Schutz darunterliegender, im wesentlichen metallischer oder reduzierte (niederwertige) Metalloxide enthaltender Schichten (B) dient.

Die Schicht (C) kann aus niedrigbrechenden oder hochbrechenden Metalloxiden, die sowohl farblos als auch selektiv absorbierend sein können, aufgebaut sein. Geeignet sind z.B. Siliciumoxid, Siliciumoxidhydrat, Aluminiumoxid, Aluminiumoxidhydrat, Zinnoxid, Titandioxid, Zirkonoxid, Eisen(III)oxid und Chrom(III)oxid, wobei Siliciumoxid und Aluminiumoxid bevorzugt sind.

Die Schicht (C) kann auch eine durch Gasphasenpassivierung zu erhaltende, phosphat-, chromat- und/oder vanadathaltige oder auch phosphat- und aminoalkylgruppenhaltige SiO₂-haltige Schicht sein, die insbesondere auch den Einsatz der eine weitgehend metallische Schicht (B) aufweisenden, erfindungsgemäßen Glanzpigmente in Wasserbasislacken oder anderen wäßrigen Systemen ermöglicht.

Die geometrische Dicke der Schicht (C) beträgt im allgemeinen etwa 1 bis 400 nm, vorzugsweise 5 bis 250 nm.

Die erfindungsgemäßen Glanzpigmente zeichmen sich durch den gleichmäßigen, homogenen und filmartigen Aufbau ihrer interferenzfähigen Beschichtung aus, welche die Substratplättchen allseitig umhüllt. Sie zeigen ein sehr kräftiges, extrem winkelabhängiges Farbenspiel mit neuen Farbflops und hoher Brillanz, das gezielt an die jeweiligen koloristischen Anforderungen angepaßt werden kann.

Die Herstellung der erfindungsgemäßen Glanzpigmente kann vorteilhaft erfolgen, indem die bevorzugte, Farbmittel enthaltende Beschichtung (A) naßchemisch durch hydrolytische Zersetzung organischer oder anorganischer Metallverbindungen in Gegenwart des Farbmittels auf die Substratteiichen aufgebracht wird und die Beschichtung (B) (sowie gewünschtenfalls auch die Beschichtung (C)) anschließend ebenfalls naßchemisch oder vorzugsweise über Gasphasenzersetzung flüchtiger Metallverbindungen (chemical vapor deposition, CVD) abgeschieden wird.

Je nach der gewünschten Verteilung des Farbmittels in der Beschichtung (A) geht man im ersten Beschichungsschritt zweckmäßigerweise wie folgt vor:

Soll das Farbmittel im wesentlichen homogen in der Beschichtung (A) verteilt sein, dosiert man die zu zersetzende Metallverbindung und das Farbmittel (gegebenenfalls als Lösung) gleichzeitig in die Suspension der Substratteilchen. Soll das Farbmittel im oberen Teil der Beschichtung (A) angereichert sein, dosiert man zunächst nur die zu zersetzende Metallverbindung zu und beginnt die Farbmitteldosierung erst, wenn bereits ausreichend Schichtmaterial (A) auf den Substratteilchen abgeschieden ist. Soll das Farbmittel umgekehrt im unteren Teil der Beschichtung (A) angereichert sein, so legt man die Substratteilchen und das Farbmittel vor und dosiert dann die zu zersetzende Metallverbindung zu. Die Konzentration an freiem Farbmittel in der Pigmentsuspension geht dann mit zunehmender Beschichtung zurück.

Bei der von organischen Metallverbindungen ausgehenden, insbesondere bei metallischen Substratteilchen bevorzugten Variante zur Abscheidung der Beschichtung (A) kann man vorteilhaft gemäß dem in der EP-A-668 329 beschriebenen Verfahren vorgehen, bei dem organische Silicium- und/oder Aluminiumverbindungen, bei welchen die organischen Reste über Sauerstoffatome an die Metalle gebunden sind, in Gegenwart der Substratteilchen und eines organischen Lösungsmittels, in welchem die Metallverbindungen löslich sind und welches mit Wasser mischbar ist, hydrolysiert werden.

Die bevorzugte Ausführungsform besteht dabei in der Hydrolyse der Metallalkoholate (insbesondere Tetraethoxysilan und Aluminiumtriisopropanolat) in Gegenwart eines Alkohols (insbesondere Isopropanol oder Ethanol) und von wäßrigem Ammoniak als Katalysator.

Verfahrenstechnisch geht man dabei vorzugsweise so vor, daß man Substratteilchen, Alkohol, Wasser und Ammoniak vorlegt, diese Mischung unter Rühren auf 40 bis 80°C, insbesondere 60 bis 70°C, erhitzt und eine Lösung des Metallalkoholats in Alkohol kontinuierlich zudosiert. Das Farbmittel gibt man dabei, wie oben beschrieben, abhängig von der gewünschten Einlagerung in die Schicht (A) (homogen oder bereichsweise angereichert) zu. Nach einer Nachrührzeit von meist etwa 1 bis 15 h kühlt man die Mischung auf Raumtemperatur ab und isoliert das beschichtete Pigment durch Abfiltrieren und Trocknen.

Siliciumoxid(hydrat)beschichtungen (A) können vorteilhaft auch ausgehend von Alkalimetallsilikaten, insbesondere von Natronwasserglas, erzeugt werden.

Man geht dabei, wie in der älteren deutschen Patentanmeldung 198 08 657.1 beschrieben, zweckmäßigerweise so vor, daß man die (vorzugsweise nichtmetallischen) Substratteilchen in Wasser suspendiert, die Suspension auf etwa 20 bis 100°C, bevorzugt 40 bis 80°C, erhitzt, mit einer Base (insbesondere einer Alkalimetallhydroxidlösung wie Kalilauge oder Natronlauge) oder einer Säure (z.B. Salpetersäure) einen pH-Wert von in der Regel 4 bis 9, vorzugsweise 6,5 bis 8,5, insbesondere etwa 7,5, einstellt und die Alkalimetallsilikatlösung unter gleichzeitiger Zugabe einer wäßrigen anorganischen Säure zur Konstanthaltung des pH-Wertes zudosiert. Auch hier gibt man das Farbmittel abhängig von dem gewünschten Beschichtungsergebnis zu (legt es mit den Substratteilchen vor, dosiert es gleichzeitig mit dem Alkalimetallsilikat oder verspätet zu). Gegebenenfalls rührt man man wenige min bis zu 2 h nach.

Die erfindungsgemäßen Beschichtungen (B) (und gegebenenfalls (C)) können vorteilhaft nach dem CVD-Verfahren oder ebenfalls naßchemisch abgeschieden werden.

Bei der CVD-Variante werden als Ausgangsverbindungen flüchtige Metallverbindungen, insbesondere Metallcarbonyle, Metallalkoholate, Metallorganyle oder Metallhalogenide, eingesetzt, die im Temperaturbereich von etwa 100 bis 600°C in Gegenwart von Inertgas, Luft oder Wasserdampf inert, oxidativ bzw. hydrolytisch zu sich auf den Pigmentteilchen abscheidenden Metall- oder Metalloxidfilmen zersetzbar sind, welche anschließend gegebenenfalls durch Umsetzung mit scinwefelhalcigen Verbindungen, oder anmoniakhaltigen Gasen in Metallsulfid- bzw. Metallnitridfilme umgewandelt werden können.

Die naßchemische Variante eignet sich vor allem zur Erzeugung von Metalloxidbeschichtungen (B), insbesondere von Titandioxidschichten, und basiert vorzugsweise auf der hydrolytischen Zersetzung geeigneter Metallsalze oder -alkoholate.

Beide Verfahrensvarianten sind hinreichend bekannt. Einzelheiten können der EP-A-753 545 und der älteren deutschen Patentanmeldung 198 08 657.1 entnommen werden.

Die erfindungsgemäßen Glanzpigmente eignen sich vorteilhaft für viele Zwecke, wie zur Einfärbung von Gläsern, keramischen Produkter, Zubereitungen der dekorativen Kosmetik und insbesondere von Lacken, Tinten, Druckfarben, auch Sicherh eitsdruckfarben, und Kunststoffen. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Pigmente auch vorteilhaft in Abmischung mit transparenten und deckenden Weiß-, Bunt- und Schwarzpigmenten sowie auch herkömmlichen Glanzpigmenten auf der Basis von metalloxidbeschichteten Glimmer- und Metallpigmenten und bekannten goniochromacischen Glanzpigmenten verwenden.

### Beispiele

### Herstellung von erfindungsgemäßen Glanzpigmenten

### Beispiel 1

a) 120 g einer 65 gew.-%igen Aluminiumpaste (mittlere Teilchengröße ca. 16 µm; Alpate® 7670 NS, Alcan Toyo) wurden in 1,5 l Isopropanol aufgeschlämmt. Nach 15-minütigem Rühren wurden nacheinander 160 g einer 25 gew.-%igen Lösung des Farbstoffs der Formel I' (CuPc: Kupferphthalocyanin) 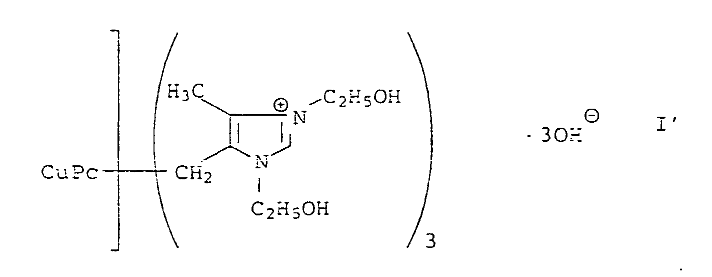 in 3 gew.-%iger wäßriger Essigsäure, 400 ml Wasser und 40 ml einer 25 gew.-%igen wäßrigen Ammoniaklösung zugegeben. Unter gleichzeitigem Erhitzen auf 60°C wurde mit der parallelen Zudosierung eines Gemisches aus 680 g Tetraethoxysilan und 680 g Isopropanol sowie von 125 ml einer 2,5 gew.-%igen wäßrigen Ammoniaklösung begonnen (Dosierdauer 24 h). Nach einer Nachrührzeit von 2 h und Abkühlen der Suspension auf Raumtemperatur wurde das Produkt abfiltriert, mit Isopropanol gewaschen und bei 70°C getrocknet.
   Es wurden 310 g eines intensiv türkisblauen Pigments erhalten.
b) 200 g des in Schritt a) erhaltenen Pigments wurden anschließend in einem Wirbelschichtreaktor unter Fluidisierung mit insgesamt 1000 l/h Stickstoff auf 200°C erhitzt. Dann wurden 40 g Molybdänhexacarbonyl mit einem Teil der Wirbelgase (4001/h) aus einer auf 80°C temperierten Vorlage in ca. 8 h in den Reaktor überführt, wo sie zu sich auf den mit (A) belegten Pigmentteilchen abscheidendem Molybdän und Kohlenmonoxid zersetzt wurden. Nach beendeter Molybdänabscheidung wurde den Wirbelgasen beim Abkühlen des Reaktors zur Passivierung der Molybdänoberfläche etwas Luft zugesetzt.

Das erhaltene Pigment hatte einen Siliciumgehalt von 24,2 Gew.-%, einen Kohlenstoffgehalt von 8,9 Gew.-% und einen Molybdängehalt von 4,7 Gew.-%. Bei Applikation zeigte es in Aufsicht eine kräftige, blaustichig grüne Interferenzfarbe, die bei zunehmender Schrägsicht nach Blau abkippte.

### Beispiel 2

a) Schritt a) von Beispiel 1 wurde viermal wiederholt, um insgesamt 1,2 kg des mit blauen Farbstoff enthaltendem Siliciumdioxid beschichteten Aluminiumpigments herzustellen.
b) 1000 g des in Schritt a) erhaltenen Pigments wurden anschließend in einem Wirbelschichtreaktor unter Fluidisierung mit insgesamt 1950 l/h Stickstoff auf 200°C erhitzt. Dann wurde ein Teil der Wirbelgase (300 l/h) über eine auf 30°C temperierte Vorlage mit 150 g Eisenpentacarbonyl in den Reaktor geleiten, ein weiterer Teil der Wirbelgase (150 l/h) wurde mit Wasserdampf (60 ml/h Wasser) gesättigt. Durch eine weitere Reaktoröffnung wurden zusätzlich 300 l/h Luft eingetragen. Nach ca. 8 h war das gesamte Carbonyl in den Reaktor überführt und zu sich auf den mit (A) belegten Pigmentteilchen abscheidendem α-Fe₂O₃ zersetzt.

Das erhaltene Pigment hatte einen Siliciumgehalt von 25,5 Gew.-%, einen Kohlenstoffgehalt von 8,9 Gew.-% und einen Eisengehalt von 2,9 Gew.-%. Bei Applikation zeigte es in Aufsicht eine kräftige, gelbstichig grüne Interferenzfarbe, die bei zunehmender Schrägsicht in ein blaustichiges Grün abkippte.

### Beispiel 3

a) Schritt a) von Beispiel 1 wurde viermal wiederholt, um insgesamt 1,2 kg des mit blauen Farbstoff enthaltendem Siliciumdioxid beschichteten Aluminiumpigments herzustellen.
b) 1000 g des in Schritt a) erhaltenen Pigments wurden anschließend in einem Wirbelschichtreaktor unter Fluidisierung mit insgesamt 1800 l/h Stickstoff auf 200°C erhitzt. Dann wurde ein Teil der Wirbelgase (400 l/h) über eine auf 50°C temperierte Wasservorlage und ein weiterer Teil der wirbelgase (400 l/h) über eine auf 120°C temperierte Vorlage mit einem Gemisch von Titantetraethanolat und Titantetraisopropanolat im Molverhältnis 1:1 (Titanat IPET, Fa. Hüls) geleitet. In ca. 8 h wurden so 150 ml des Titantetraalkoholatgemischs portionsweise in den Reaktor überführt und zu sich auf den mit (A) belegten Pigmentteilchen abscheidendem TiO₂ zersetzt.

Das erhaltene Pigment hatte einen Siliciumgehalt von 25,8 Gew.-%, einen Kohlenstoffgehalt von 8,9 Gew.-% und einen Titangehalt von 2,2 Gew.-%. Bei Applikation zeigte es in Aufsicht einen kräftigen Türkisfarbton, der bei zunehmender Schrägsicht nach blau wechselte.

### Beispiel 4

a) 130 g titandioxidbeschichtetes Glimmerpigment (Iriodin® Sterling Silber 103; Merck) wurden in 1 1 Wasser aufgeschlämmt. Nach 15-minütigem Rühren wurden 250 g einer 25 gew.-%igen Lösung des Farbstoffs der Formel I' aus Beispiel 1 in 3 gew.-%iger wäßriger Essigsäure zugegeben. Nach Erhitzen der Suspension auf 75°C wurden 1540 ml Natronwasserglaslösung (68 g Si/l) in ca. 48 h zugegeben, wobei der pH-Wert der Suspension durch gleichzeitige Zugabe von 416 ml 20 gew.-%iger Salpetersäure bei 8,8 gehalten wurde. Nach einer Nachrührzeit von 1 h und Abkühlen auf Raumtemperatur wurde das Produkt abfiltriert, mit Wasser und mit Ethanol gewaschen und anschließend bei 75°C unter Vakuum getrocknet.
   Es wurden 429 g eines blauen Pigments erhalten.
   Schritt a) wurde noch zweimal wiederholt, um insgesamt etwa 1,2 kg des mit blauen Farbstoff enthaltendem Siliciumdioxid beschichteten Glimmerpigments herzustellen.
b) 1000 g des in Schritt a) erhaltenen Pigments wurden anschließend analog Beispiel 3b) mit Titandioxid belegt.

Das erhaltene Pigment hatte einen Siliciumgehalt von 27,3 Gew.-%, einen Kohlenstoffgehalt von 8,0 Gew.-% und einen Titangehalt von 6,9 Gew.-%. Bei Applikation zeigte es in Aufsicht eine kräftige, grünstichig blaue Interferenzfarbe, die bei zunehmender Schrägsicht nach dunkelblau wechselte.

## Patentansprüche

1. Glanzpigmente auf der Basis von mehrfach beschichteten, reflektierenden, plättchenförmigen Substraten, die metallische oder hochbrechende nichtmetallische Plättchen mit einem Brechungsindex ≥ 2, die jeweils bereits mit einer hochbrechenden Schicht belegt sein können, oder niedrigbrechende nichtmetallische Plättchen, die bereits mit einer hochbrechenden Schicht belegt sind, umfassen und mindestens ein Schichtpaket aus
A) zunächst einer niedrigbrechenden, sichtbares Licht selektiv absorbierenden Beschichtung mit einem Brechungsindex n ≤ 1, 8 und
B) anschließend einer reflektierenden, für sichtbares Licht zumindest teilweise durchlässigen Beschichtung
sowie gewünschtenfalls zusätzlich
C) eine äußere Schutzschicht
aufweisen.

2. Glanzpigmente nach Anspruch 1, bei denen die Beschichtung (A) ein farbloses niedrigbrechendes Material umfaßt, in das ein oder mehrere selektiv absorbierende Farbmittel eingelagert sind.

3. Glanzpigmente nach Anspruch 2, bei denen das Farbmittel im wesentlichen homogen in der Beschichtung (A) verteilt ist oder in einem Teilbereich der Beschichtung (A) angereichert ist.

4. Glanzpigmente nach den Ansprüchen 1 bis 3, bei denen die Beschichtung (A) einen kationischen Farbstoff enthält.

5. Glanzpigmente nach den Ansprüchen 1 bis 5, bei denen die Beschichtung (A) eine geometrische Dicke von 100 bis 800 nm aufweist.

6. Glanzpigmente nach den Ansprüchen 1 bis 6, bei denen die Beschichtung (B) hochbrechende Materialien, die sichtbares Licht nicht absorbieren oder selektiv oder nichtselektiv ab-sorbieren, oder niedrigbrechende Materialien, die sichtbares Licht stark absorbieren, umfaßt.

7. Glanzpigmente nach den Ansprüchen 1 bis 7, bei denen die Beschichtung (B) einen Brechungsindex n ≥ 2 und/oder eine Absorptionskonstante k ≥ 4 aufweist.

8. Verwendung von Glanzpigmenten gemäß den Ansprüchen 1 bis 8 zur Einfärbung von Lacken, Druckfarben, Tinten, Kunststoffen, Gläsern, keramischen Produkten und Zubereitungen der dekorativen Kosmetik.

## Claims

1. Luster pigments based on multiply coated reflecting platelet-shaped substrates which comprise metallic or high refractive nonmetallic platelets having a refractive index ≥ 2, which may each already be coated with a high refractive layer, or low refractive nonmetallic platelets which are already coated with a high refractive layer and comprising at least one layer packet comprising
A) firstly a low refractive coating which selectively absorbs visible light and has a refractive index n ≤ 1.8 and
B) then a reflective coating which is at least partially transparent to visible light
and also, if desired,
C) an outer protective layer.

2. The luster pigments according to claim 1, wherein the coating (A) comprises a colorless low refractive material embedding one or more selectively absorbing colorants.

3. The luster pigments according to claim 2, wherein the colorant is substantially homogeneously dispersed in the coating (A) or concentrated in a region of coating (A).

4. The luster pigments according to any of claims 1 to 3, wherein the coating (A) includes a cationic dye.

5. The luster pigments according to any of claims 1 to 4, wherein the coating (A) has a geometric thickness of from 100 to 800 nm.

6. The luster pigments according to any of claims 1 to 5, wherein the coating (B) comprises high refractive materials which absorb visible light not at all or selectively or nonselectively or low refractive materials which strongly absorb visible light.

7. The luster pigments according to any of claims 1 to 6, wherein the coating (B) has a refractive index n ≥ 2 and/or an absorption constant k ≥ 4.

8. The use of luster pigments as set forth in any of claims 1 to 7 for coloring paints, inks, including printing inks, plastics, glasses, ceramic products and decorative cosmetic preparations.

## Revendications

1. Pigments brillants à base de substrats en forme de paillettes, réfléchissants, enduits à plusieurs reprises, qui comportent des paillettes métalliques ou non métalliques hautement réfringentes présentant un indice de réfraction ≥2, qui chacune peuvent être déjà recouvertes d'une couche hautement réfringente, ou des paillettes non métalliques faiblement réfringentes, qui sont déjà recouvertes d'une couche hautement réfringente, et présentent au moins un paquet de couches à base
A) tout d'abord d'une enduction faiblement réfringente, absorbant de manière sélective la lumière visible et présentant un indice de réfraction n≤1, 8, et
B) ensuite d'une enduction réfléchissante, au moins partiellement transparente pour la lumière visible,
ainsi qu'éventuellement en supplément
C) d'une couche de protection externe.

2. Pigments brillants suivant la revendication 1, dans lesquels l'enduction (A) comporte une matière faiblement réfringente, incolore, dans laquelle sont incorporés un ou plusieurs colorants absorbant de manière sélective.

3. Pigments brillants suivant la revendication 2, dans lesquels le colorant est réparti de manière sensiblement homogène dans l'enduction (A) ou est enrichi dans une zone partielle de l'enduction (A).

4. Pigments brillants suivant les revendications 1 à 3, dans lesquels l'enduction (A) contient un colorant cationique.

5. Pigments brillants suivant les revendications 1 à 4, dans lesquels l'enduction (A) présente une épaisseur géométrique de 100 à 800 nm.

6. Pigments brillants suivant les revendications 1 à 5, dans lesquels l'enduction (B) comporte des matières hautement réfringentes, qui n'absorbent pas de lumière visible ou l'absorbent de manière sélective ou non sélective, ou des matières faiblement réfringentes, qui absorbent fortement la lumière visible.

7. Pigments brillants suivant les revendications 1 à 6, dans lesquels l'enduction (B) présente un indice de réfraction n≥2 et/ou une constante d'absorption k≥4.

8. Utilisation de pigments brillants suivant les revendications 1 à 7, pour colorer des vernis, des couleurs d'imprimerie, des encres, des substances synthétiques, des verres, des produits céramiques et des compositions de la cosmétique décorative.
